# EUROPEAN PATENT APPLICATION

(11) **EP 1 946 717 A1**
(43) Date of publication of application: **23.07.2008**
(21) Application number: 08250219.6
(22) Date of filing: 17.01.2008
(51) Int. Cl.: A61B 18/18

(54) **Catheter with microphone**

(30) Priority: 18.01.2007 US 624280
(71) Applicant: Biosense Webster, Inc., Diamond Bar, CA 91765 (US)
(72) Inventor: Govari, Assaf, Haifa 34400 (IL); Ephrath, Yaron, Karkur 35170 (IL); Altmann, Andres Claudio, Haifa 34614 (IL); Schwartz, Yitzhack, Haifa 34606 (IL); Dmitri, Model, Haifa 32990 (IL)
(74) Representative: Small, Gary James

(57) **Abstract**

An invasive medical probe includes an insertion tube, having a distal end for insertion in a body cavity of a patient. A metallic material is disposed over the distal tip of the insertion tube and extends from the tip so as to cover a portion of the distal end. An acoustic sensor is contained entirely within the portion of the distal end of the insertion tube that is covered by the metallic material.

## Description

### FIELD OF THE INVENTION

The present invention relates generally to invasive medical devices, and specifically to the use of audio sensors in probes, such as catheters, that are inserted into the body of a patient.

### BACKGROUND OF THE INVENTION

The use of an audio sensor inside a cardiac catheter has been suggested in the patent literature. For example, U.S. Patent 2,949,910, whose disclosure is incorporated herein by reference, describes a "phonocardiac" catheter containing an electro-acoustical transducer element in order to transduce various sounds within the heart. The catheter is made from plastic tubing, which is sealed at its end by a slug of plastic material.

U.S. Patent 6,709,432, whose disclosure is incorporated herein by reference, describes the use of acoustical monitoring to detect instability of an ablation electrode and/or crater formation during tissue ablation. An acoustic ablation catheter includes an ablation electrode and a detection sensor, which is preferably an acoustic sensing element, such as a contact microphone. In one embodiment, the microphone is acoustically coupled to the inner surface of the ablation electrode. The microphone produces a transducer signal that is representative of acoustical energy detected during ablation, including a cardiac-generated component representative of cardiac-based sounds (such as heart valves closing and blood flow) and an ablation component representative of sounds caused by the ablation procedure (such as cell explosion and the catheter hitting the endocardial wall). Sound analysis is used to remove the cardiac-generated component from the transducer signal in order to obtain an ablation sound spectrum.

Kotini et al. describe experiments in which acoustical signals were used to monitor tissue ablation in "Detection of Microbubble Formation During Radiofrequency Ablation Using Phonocardiograph," Europace 8 (2006), pages 333-335, which is incorporated herein by reference. The authors found that explosive "pops," which occur due to excessive tissue heating, may be preceded by characteristic acoustic signatures of microbubble formation.

### SUMMARY OF THE INVENTION

The inventors have found that when an acoustic sensor in the distal end of a probe is entirely contained within a metallic material covering the distal end of the probe, the acoustic sensor receives sounds almost exclusively from the tissue with which the metallic material is in contact. Ambient sounds (such as those made by the heart valves and blood flowing within the heart) are attenuated very strongly by the metallic material. Therefore, when the probe is used in an ablation procedure, for example, the acoustic sensor may be used to monitor sounds caused by the ablation itself without requiring sound analysis to remove the ambient sounds.

There is therefore provided, in accordance with an embodiment of the present invention, an invasive medical probe, including:
an insertion tube, having a distal end for insertion in a body cavity of a patient, the distal end terminating in a distal tip;
a metallic material disposed over the distal tip of the insertion tube and extending from the tip so as to cover a portion of the distal end; and
an acoustic sensor, contained entirely within the portion of the distal end of the insertion tube that is covered by the metallic material.

In some embodiments, the insertion tube includes a flexible insulating material, and the metallic material is configured to serve as an electrode. Typically, the insertion tube has a proximal end, and contains cabling running from the proximal end to the distal end and coupled to supply radio frequency (RF) energy to the metallic material for application to tissue with which the metallic material is in contact within the body cavity. The insertion tube may be configured for percutaneous insertion through a blood vessel into a heart chamber of the patient.

In a disclosed embodiment, the portion of the distal end that is covered by the metallic material extends proximally from the distal tip over at least 2 mm of the insertion tube.

There is also provided, in accordance with an embodiment of the present invention, apparatus for medical treatment, including:
an invasive medical probe, which includes:
   an insertion tube, having a proximal end and a distal end for insertion in a body cavity of a patient, the distal end terminating in a distal tip;
   a metallic material disposed over the distal tip of the insertion tube and extending from the tip so as to cover a portion of the distal end;
   an acoustic sensor, contained entirely within the portion of the distal end of the insertion tube that is covered by the metallic material; and
   cabling, running from the proximal end to the distal end within the insertion tube and coupled to the metallic material and to the acoustic sensor at the distal end;
a radio frequency (RF) generator, which is coupled to the cabling to as to supply RF energy to the metallic material for ablation of tissue with which the metallic material is in contact within the body cavity; and
an acoustic processor, which is coupled to the cabling so as to receive and process an audio signal generated by the acoustic sensor in order to provide an indication of the ablation.

In a disclosed embodiment, the insertion tube is configured for percutaneous insertion through a blood vessel into a chamber of a heart of the patient, and the acoustic processor is configured to provide the indication of the ablation without filtering the audio signal to remove ambient sounds generated by normal action of the heart. Typically, the indication of the ablation is indicative of a phenomenon selected from a group of phenomena consisting of cavitation in the tissue and intermittent contact between the distal tip of the insertion tube and the tissue.
In one embodiment, the indication of the ablation that is generated by the acoustic processor includes an amplified audio signal, and the apparatus includes an audio output device, which is coupled to play the amplified audio signal to an operator of the apparatus.

There is additionally provided, in accordance with an embodiment of the present invention, a method for medical treatment, including:
inserting a probe into a body cavity of a patient, the probe including an insertion tube having a proximal end and a distal end terminating in a distal tip, with a metallic material disposed over the distal tip of the insertion tube and extending from the tip so as to cover a portion of the distal end, and an acoustic sensor contained entirely within the portion of the distal end of the insertion tube that is covered by the metallic material;
bringing the distal tip of the probe into contact with tissue within the body cavity;
applying radio frequency (RF) energy to the metallic material so as to ablate the tissue with which the distal tip is in contact; and
receiving and processing an audio signal generated by the acoustic sensor in order to provide an indication of ablation of the tissue.

The present invention will be more fully understood from the following detailed description of the embodiments thereof, taken together with the drawings in which:

### BRIEF DESCRIPTION OF THE DRAWINGS

Fig. 1 is a schematic, pictorial illustration of a catheter-based medical system, in accordance with an embodiment of the present invention; and
Fig. 2 is a schematic detail view showing the distal tip of a catheter in contact with myocardial tissue, in accordance with an embodiment of the present invention.

### DETAILED DESCRIPTION OF EMBODIMENTS

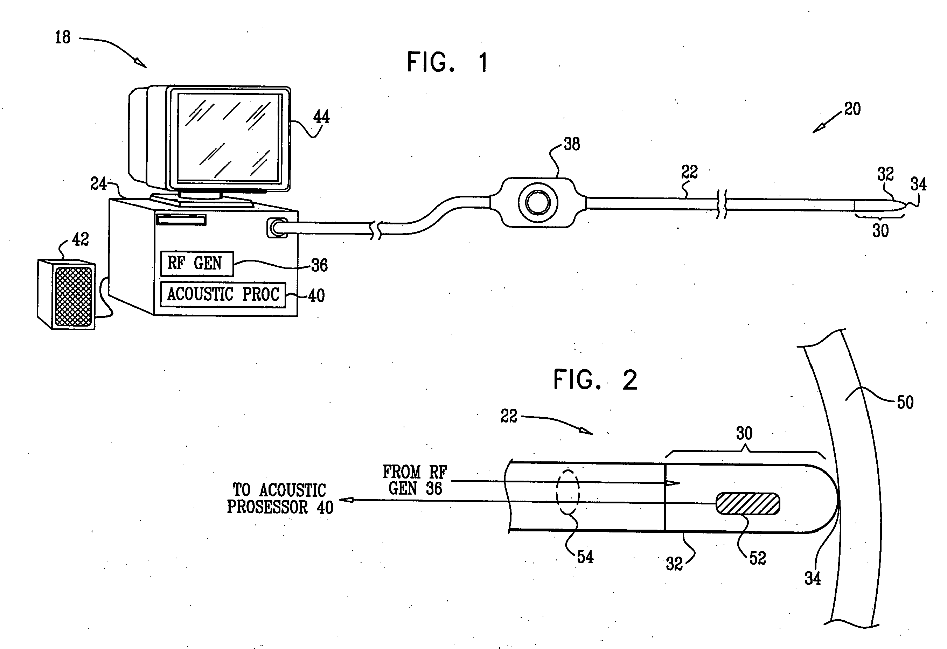

Fig. 1 is a schematic, pictorial illustration of a system 18 for cardiac catheterization, in accordance with an embodiment of the present invention. System 18 may be based, for example, on the CARTO^{™} system, produced by Biosense Webster Inc. (Diamond Bar, California). This system comprises an invasive probe in the form of a catheter 20 and a control console 24. Alternatively, the principles of the present invention may be implemented in other cardiac catheters, as well as invasive probes of other kinds.

Catheter 20 comprises an insertion tube 22 whose distal end 30 is designed to be passed through the vascular system and into a chamber of the heart. The insertion tube typically comprises a flexible, insulating material, such as Celcon^{®} or Teflon^{®}. Distal end 30 of the catheter is covered by an electrode 32, which is typically made of a metallic material, such as a platinum/iridium alloy, and extends from a distal tip 34 of the catheter over a portion of the distal end. Alternatively, other suitable materials may be used, as will be apparent to those skilled in the art. The inventors have found that an electrode layer that is about 300 µm thick and extends from the distal tip over a length of at least 2 mm, and typically about 4 mm, in the proximal direction gives good results in terms of both ablation and acoustic shielding, but greater or smaller' thicknesses and lengths may also be used.

Catheter 20 is typically connected by a suitable connector at its proximal end to console 24. The console comprises a radio frequency (RF) generator 36, which supplies high-frequency electrical energy via the catheter to electrode 32. The operator of system 18 typically uses controls on a handle 38 of the catheter to manipulate distal end 30 within a body cavity (such as a chamber of the heart), in order to bring electrode 32 into contact with target tissue. The electrical energy carried by the electrode is used to ablate the target tissue. Ablation of this sort in the heart is typically used, as is known in the art, for treatment of arrhythmias. Alternatively, the catheter may be used *mutatis mutandis,* for other therapeutic and/or diagnostic purposes in the heart or in other body organs.

Fig. 2 is a schematic detail view showing distal tip 34 of insertion tube 22 in contact with myocardial tissue 50 within the heart, in accordance with an embodiment of the present invention. As shown in this figure, distal end 30 contains a miniature acoustic sensor 52, such as a microphone. Suitable sensors for this purpose include the DigiSiMic microphone (model TC100Z21A) or SiMic microphone (model TC200Z03F), with dimensions of 2.3 x 1.6 x 0.86 mm, manufactured by Sonion (Roskilde, Denmark); or the Omni-directional Electret Microphone (model FG-3329), with dimensions of 2.59 mm OD x 2.59 mm height, manufactured by Knowles Acoustics (Itasca, Illinois). Alternatively, any other suitable type of acoustic sensor may be used, such as those described in the patents cited above.

Sensor 52 is connected by cabling 54 running through insertion tube 22 to an acoustic processor 40 in console 24 (Fig. 1). (In the embodiment shown in Fig. 2, the cabling comprises multiple conductors, including wires used to convey electrical energy from RF generator 36 to electrode 32.) The acoustic processor typically comprises analog amplification circuits, and may comprise additional analog and/or digital signal processing components for filtering and possibly analyzing the audio signal that is output by sensor 52. Alternatively, certain amplification, filtering, and even digital processing components may be contained in catheter 20 along with the acoustic sensor.

During myocardial ablation, catheter 20 should be carefully controlled in order to achieve therapeutic goals and avoid excessive injury to the heart tissue. For example, if the temperature in the vicinity of the ablation site increases too much, the gas bubbles formed by the resulting cavitation may explode, blowing a crater or even a hole through the heart wall. As another example, if the catheter is not firmly pressed against the target tissue, the RF ablation energy will dissipate in the surrounding blood and will not give the desired ablation lesion.

Audio signals picked up by sensor 52 can provide an early indication of problematic phenomena that may occur in ablation therapy. These signals are therefore amplified and, optionally, filtered by acoustic processor 40, which then passes the signals to an audio output device 42, such as a speaker or headphones. For example, cavitation in the vicinity of the catheter tip causes a hissing sound, evidently due to bubble formation. Upon hearing such a hiss (or an increase in the intensity of the hiss), the operator may choose to reduce the electrical power supplied by RF generator 36 and thus avoid excessive cavitation that may lead to explosion. As another example, if distal tip 34 is not firmly pressed against the myocardium, sensor 52 will pick up a periodic bumping sound caused by intermittent contact between the tip and the myocardium. In this case, the operator may advance the catheter so that proper contact is achieved, whereupon the bumping sound should disappear or at least decrease substantially.

As noted above, the metallic material of electrode 32 filters out ambient sounds generated by normal action of the heart. Local sounds in tissue 50 with which the electrode is in contact, however, reach sensor 52 without such strong attenuation. Thus, because the sensor is entirely contained within the portion of distal.end 30 that is covered by electrode 32, there is generally no need for acoustic processor 40 to filter out the ambient heart sounds in order to provide the indication of ablation based on the local sounds. Avoiding the need for this sort of filtering simplifies the construction and programming of the acoustic processor and enhances the fidelity of the sounds that are played by audio output device 42.

Additionally or alternatively, acoustic processor 40 may automatically analyze the audio signals that are output by sensor 52 in order to detect local sounds, such as hissing and bumping. For example, processor 40 may perform automated analysis based on the sound characteristics described in the above-mentioned article by Kotini et al. In this case, if the acoustic processor detects such sounds with volume above a certain threshold level, it may output an audible and/or visual alarm, on a display 44, for example.

It will be appreciated that the embodiments described above are cited by way of example, and that the present invention is not limited to what has been particularly shown and described hereinabove. Rather, the scope of the present invention includes both combinations and subcombinations of the various features described hereinabove, as well as variations and modifications thereof which would occur to persons skilled in the art upon reading the foregoing description and which are not disclosed in the prior art.

## Claims

1. An invasive medical probe, comprising:
an insertion tube, having a distal end for insertion in a body cavity of a patient, the distal end terminating in a distal tip;
a metallic material disposed over the distal tip of the insertion tube and extending from the tip so as to cover a portion of the distal end; and
an acoustic sensor, contained entirely within the portion of the distal end of the insertion tube that is covered by the metallic material.

2. The probe according to claim 1, wherein the insertion tube comprises a flexible insulating material, and wherein the metallic material is configured to serve as an electrode.

3. The probe according to claim 2, wherein the insertion tube has a proximal end, and contains cabling running from the proximal end to the distal end and coupled to supply radio frequency (RF) energy to the metallic material for application to tissue with which the metallic material is in contact within the body cavity.

4. The probe according to claim 1, wherein the insertion tube is configured for percutaneous insertion through a blood vessel into a heart chamber of the patient.

5. The probe according to claim 1, wherein the portion of the distal end that is covered by the metallic material extends proximally from the distal tip over at least 2 mm of the insertion tube.

6. Apparatus for medical treatment, comprising:
an invasive medical probe, which comprises:
an insertion tube, having a proximal end and a distal end for insertion in a body cavity of a patient, the distal end terminating in a distal tip;
a metallic material disposed over the distal tip of the insertion tube and extending from the tip so as to cover a portion of the distal end;
an acoustic sensor, contained entirely within the portion of the distal end of the insertion tube that is covered by the metallic material; and
cabling, running from the proximal end to the distal end within the insertion tube and coupled to the metallic material and to the acoustic sensor at the distal end;
a radio frequency (RF) generator, which is coupled to the cabling to as to supply RF energy to the metallic material for ablation of tissue with which the metallic material is in contact within the body cavity; and
an acoustic processor, which is coupled to the cabling so as to receive and process an audio signal generated by the acoustic sensor in order to provide an indication of the ablation.

7. The apparatus according to claim 6, wherein the insertion tube is configured for percutaneous insertion through a blood vessel into a chamber of a heart of the patient.

8. The apparatus according to claim 7, wherein the acoustic processor is configured to provide the indication of the ablation without filtering the audio signal to remove ambient sounds generated by normal action of the heart.

9. The apparatus according to claim 6, wherein the indication of the ablation is indicative of a phenomenon selected from a group of phenomena consisting of cavitation in the tissue and intermittent contact between the distal tip of the insertion tube and the tissue.

10. The apparatus according to claim 6, wherein the indication of the ablation that is generated by the acoustic processor comprises an amplified audio signal, and comprising an audio output device, which is coupled to play the amplified audio signal to an operator of the apparatus.

11. A method for medical treatment, comprising:
inserting a probe into a body cavity of a patient, the probe comprising an insertion tube having a proximal end and a distal end terminating in a distal tip, with a metallic material disposed over the distal tip of the insertion tube and extending from the tip so as to cover a portion of the distal end, and an acoustic sensor contained entirely within the portion of the distal end of the insertion tube that is covered by the metallic material;
bringing the distal tip of the probe into contact with tissue within the body cavity;
applying radio frequency (RF) energy to the metallic material so as to ablate the tissue with which the distal tip is in contact; and
receiving and processing an audio signal generated by the acoustic sensor in order to provide an indication of ablation of the tissue.

12. The method according to claim 11, wherein inserting the probe comprises passing the probe percutaneously through a blood vessel into a heart chamber of the patient.

13. The method according to claim 12, wherein processing the audio signal comprises providing the indication of the ablation without filtering the audio signal to remove ambient sounds generated by normal action of the heart.

14. The method according to claim 11, wherein the indication of the ablation is indicative of a phenomenon selected from a group of phenomena consisting of cavitation in the tissue and intermittent contact between the distal tip of the insertion tube and the tissue.

15. The method according to claim 11, wherein processing the audio signal comprises amplifying and outputting the audio signal to an audio output device, so as to play the amplified audio signal to an operator of the probe.
